# EUROPEAN PATENT APPLICATION

(11) **EP 2 839 779 A1**
(43) Date of publication of application: **25.02.2015**
(21) Application number: 13778140.7
(22) Date of filing: 18.04.2013
(51) Int. Cl.: A61B 5/0408, A61B 5/0428, A61B 5/0452, A61B 5/0478, B60N 2/44, B60R 21/015

(54) **VEHICLE-USE SEAT**

(30) Priority: 19.04.2012 JP 2012095788
(71) Applicant: TS Tech Co., Ltd., Asaka-shi, Saitama 351-0012 (JP); Josho Gakuen Educational Foundation, Osaka 535-8585 (JP); Nihon University, Tokyo 102-8275 (JP)
(72) Inventor: SUGIYAMA, Shinji, Shioya-gun Tochigi 329-1217 (JP); NAKANO, Toyokazu, Shioya-gun Tochigi 329-1217 (JP); OHSUGA, Mieko, Osaka-shi Osaka 535-8585 (JP); KOBAYASHI, Hiroyuki, Osaka-shi Osaka 535-8585 (JP); YASUDA, Shota, Osaka-shi Osaka 535-8585 (JP); KAGEYAMA, Ichiro, Tokyo 102-8275 (JP); KURIYAGAWA, Yukiyo, Tokyo 102-8275 (JP)
(74) Representative: Grünecker, Kinkeldey, Stockmair & Schwanhäusser
(86) International application number: PCT/JP2013/061520
(87) International publication number: WO 2013/157608

(57) **Abstract**

To make it possible to stably measure heartbeats even if seated persons physically differ and to measure the heartbeats of even a seated person other than a driver. A vehicle seat includes : a seat back (104) ; and a measuring device including a plurality of sensors (20A, 20B, 20C) that are provided in the seat back (104) and that acquire body potentials of the seated person, and an arithmetic operation device that computes electrical signals relating to the detected body potentials to obtain an electrocardiographic signal of the seated person. The sensors (20A, 20B, 20C) are each capacitively coupled sensors. The sensor (20A) is arranged at a lower right position relatively to the sensor (20B) and the sensor (20C) while facing a front surface of the seat back (104) if a surface of the seat back (104) facing the seated person is assumed as the front surface. The sensor (20A) and the sensor (20C) are arranged so that a heart of the seated person is located in a zone (20AC) if the zone (20AC) is defined from the sensor (20A) and the sensor (20C).

## Description

### Technical Field

The present invention relates to a vehicle seat and relates to a vehicle seat having a function of measuring heartbeats.

### Background Art

In recent years, there has been proposed a configuration for determining whether a driver has a health problem by detecting various parameters indicative of a driver's condition with views of preventing a serious accident that possibly occurs while the driver is driving a vehicle and so as to promptly notify the driver of the occurrence of the health problem.

For example, Patent Literature 1 discloses a measuring device including a plurality of surface electrodes arranged at respective positions on which the back, a region from the waist to the buttocks, and the femoral region of the driver abut. The measuring device suppresses signal noise by allowing one of the surface electrodes to acquire the potential of the neutral point of an amplifier, and determines abnormality by appropriately detecting heartbeat signals and respiration signals of the driver.

Patent Literature 2 discloses an electrocardiographic measuring device including two sensors arbitrarily arranged in a seat back and a ground electrode arranged in a seat cushion.

Patent Literature 3 discloses a vehicle electrocardiographic measuring device including a sensor provided in a steering portion for detecting the body potential of a driver.

### Citation List

### Patent Document

Patent Document 1: JP 2007-301175 A
Patent Document 2: JP 2009-50679 A
Patent Document 3: JP 2011-24902 A

### SUMMARY OF THE INVENTION

### Problems to be Solved by the Invention

Regions where a vehicle driver contacts a seat greatly differ between a case where the driver has a small build and a case where the driver has a large build. For example, in a case of the vehicle seat disclosed in Patent Literature 1, irregularities occur in the signals obtained by the surface electrodes if drivers physically differ.

Furthermore, the electrocardiographic measuring device disclosed in Patent Literature 2 is intended to detect electrocardiographic signals using the two sensors provided in the seat back, and the sensors are not arranged depending on the physical difference. Owing to this, if the drivers physically differ, it is impossible to stably detect the electrocardiographic signals.

Moreover, in a case of the electrocardiographic measuring device disclosed in Patent Literature 3, a seat provided with this device is limited to a driver' s seat provided with a steering wheel, and a measurement target person is limited to the driver who touches the steering wheel.

The present invention has been made in view of these problems, and an object of the present invention is to make it possible to stably measure heartbeats even if seated people differ physically, and to measure even heartbeats of those other than a driver.

### Means for Solving the Problems

The problem can be solved by a vehicle seat according to the present invention. The vehicle seat includes: a seat back on which a seated person leans; and a measuring device including a plurality of sensors that are capacitively coupled sensors acquiring body potentials of the seated person in a non-contact manner, and that are provided in the seat back; and an arithmetic operation device that computes electrical signals relating to the detected body potentials to obtain an electrocardiographic signal of the seated person, the measuring device measuring heartbeats of the seated person, wherein the sensors are provided to face the seated person in a range from a lower portion to an upper portion in the seat back, and include at least three sensors of a first sensor provided in the lower portion, a second sensor provided in an intermediate portion, and a third sensor provided in the upper portion, wherein the first sensor is arranged at a lower right position of a front surface of the seat back relatively to the second sensor and the third sensor when seen from a direction facing the front surface of the seat back, and the front surface is a surface of the seat back facing the seated person, wherein the second sensor is arranged above the first sensor and below the third sensor, wherein the first sensor and the third sensor are arranged so that a heart of the seated person is located in a first zone if the first zone is defined based on a position of the first sensor and a position of the third sensor and a second zone is defined based on the position of the first sensor and a position of the second sensor, and wherein the first sensor, the second sensor, and the third sensor transmit electrical signals at least relating to the body potentials detected in the first zone and the second zone to the arithmetic operation device.

By doing so, even if seated people physically differ, it is possible to stably detect a large potential difference signal corresponding to the heart contraction of each seated person, and to measure the heartbeats of the seated person other than a driver by providing the sensors in seats other than a driver's seat. Furthermore, since the capacitively coupled sensors are provided in the seat back, the heartbeats can be measured without causing the seated person to feel inconvenience of attaching the sensor to the seated person.

It is more preferable that the second sensor is provided at the position of a height larger than a height of a center of the first zone.

By doing so, even if the seated person is a person of a small build having a small contact area, it is possible to provide a large difference signal between the detected body potentials.

Moreover, it is preferable that a length in a direction of a width between the first sensor and the third sensor is larger than a length in a direction of a width between the first sensor and the second sensor.

By so setting, it is possible to provide a large potential difference signal between the body potentials detected from the seated person of each physical constitution since the widths between the sensors are set depending on the body width of the seated person of the small build and that of the seated person of the large build.

Furthermore, it is preferable that the plurality of sensors further include a fourth sensor above the first sensor and below the third sensor, and that the first sensor, the second sensor, the third sensor, and the fourth sensor transmit the body potentials detected in the first zone, the second zone, and a third zone to the arithmetic operation device for computing the body potentials to obtain the electrocardiographic signal of the seated person if the third zone is defined based on the position of the third sensor and a position of the fourth sensor.

In this way, it is possible to stably obtain electrocardiographic signals by the arithmetic operation of the arithmetic operation device since the number of detected potential difference signals increases by increasing the number of sensors provided in the seat back.

Moreover, it is preferable that each of the first sensor, the second sensor, and the third sensor includes a detection surface having shorter sides and longer sides, and is arranged so that a direction of the longer sides is along a width direction of the seat back.

By doing so, the seat back can be downsized in the height direction.

Furthermore, it is preferable that the measuring device measures the heartbeats by selecting data having periodic amplitude corresponding to a heart contraction from among data detected by the plurality of sensors defining the zones.

By doing so, it is possible to stably measure the heartbeats based on the stable data corresponding to the heart contraction.

### Effects of the Invention

According to the present invention, even if seated people physically differ, it is possible to stably detect a large potential difference signal corresponding to the heart contraction of each seated person, and to measure the heartbeats of even the seated person other than a driver. Furthermore, since the capacitively coupled sensors are provided in the seat back, the heartbeats can be measured without causing the seated person to feel inconvenience of attaching the sensor to the seated person.

Furthermore, according to the present invention, even if the seated person is a person of a small build having a small contact area, it is possible to provide a large difference signal between the detected body potentials.

Moreover, according to the present invention, it is possible to provide a large potential difference signal between the body potentials detected from the seated person of each physical constitution since the widths between the sensors are set depending on the body width of the seated person of the small build and that of the seated person of the large build.

Furthermore, according to the present invention, it is possible to stably obtain electrocardiographic signals by the arithmetic operation of the arithmetic operation device since the number of detected potential difference signals increases by increasing the number of sensors provided in the seat back.

Moreover, according to the present invention, the seat back can be downsized in the height direction.

Furthermore, according to the present invention, it is possible to stably measure the heartbeats based on the stable data corresponding to the heart contraction.

### Brief Description of Drawings

[FIG. 1] FIG. 1 shows an overall configuration of a vehicle seat according to an embodiment.
[FIG. 2] FIG. 2 is a cross-sectional view of a seat cushion and a seat back for showing arrangement positions of sensors.
[FIG. 3] FIG. 3 is a front view of the seat back for showing the arrangement of the sensors.
[FIG. 4] FIG. 4(a) shows a sensor having a rectangular detection surface, FIG. 4 (b) shows a sensor having a chamfered rectangular detection surface, and FIG. 4(c) shows a sensor having an elliptical detection surface.
[FIG. 5] FIG. 5 shows a difference in heart height depending on a physical difference.
[FIG. 6] FIG. 6 is a schematic diagram showing a circuit configuration and a configuration of an arithmetic operation device for detecting electrocardiographic signals.
[FIG. 7] FIGS. 7(a) to 7(c) show examples of detected waveform data.
[FIG. 8] FIG. 8 is a flowchart showing a flow of a heartbeat measuring process.
[FIG. 9] FIG. 9 is a front view of a seatback for showing arrangement of sensors according to a first modification.
[FIG. 10] FIG. 10 is a front view of a seatback for showing arrangement of sensors according to a second modification.
[FIG. 11] FIG. 11 is a front view of a seatback for showing arrangement of sensors according to a third modification.
[FIG. 12] FIGS. 12(a) to 12(c) show combinations of sensors for detecting a potential difference.
[FIG. 13] FIG. 13 is a front view of a seatback for showing arrangement of sensors according to a fourth modification.
[FIG. 14] FIG. 14 is a front view of a seatback for showing arrangement of sensors according to a fifth modification.
[FIG. 15] FIG. 15 is a front view of a seatback for showing arrangement of sensors according to a sixth modification.
[FIG. 16] FIG. 16 is a schematic diagram showing a configuration of the sensors according to the sixth modification.

### DETAILED DESCRIPTION OF THE INVENTION

An embodiment of a vehicle seat according to the present invention will be specifically described hereinafter with reference to the accompanying drawings.

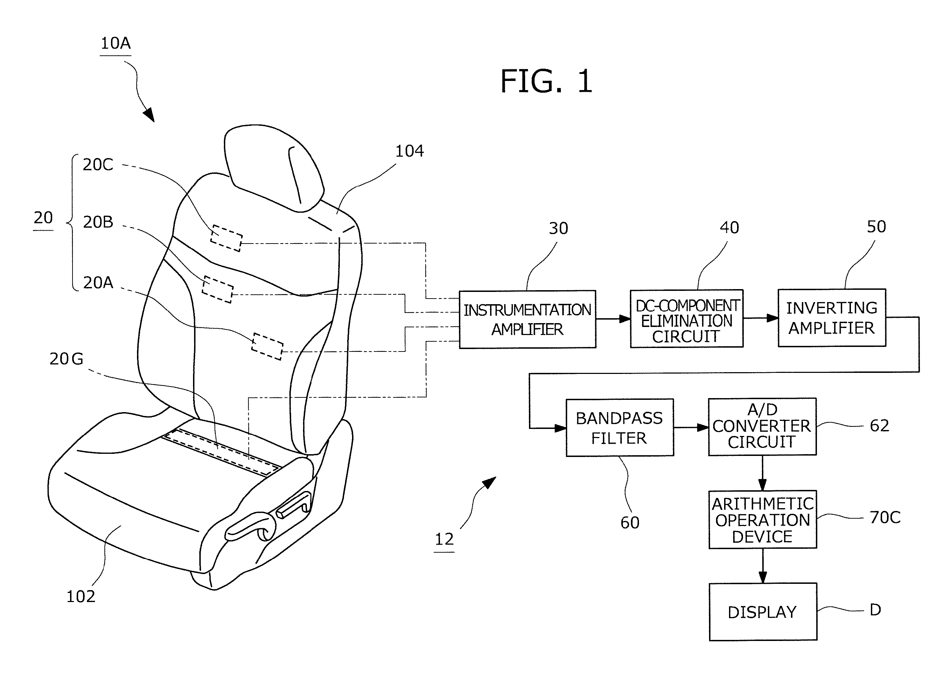

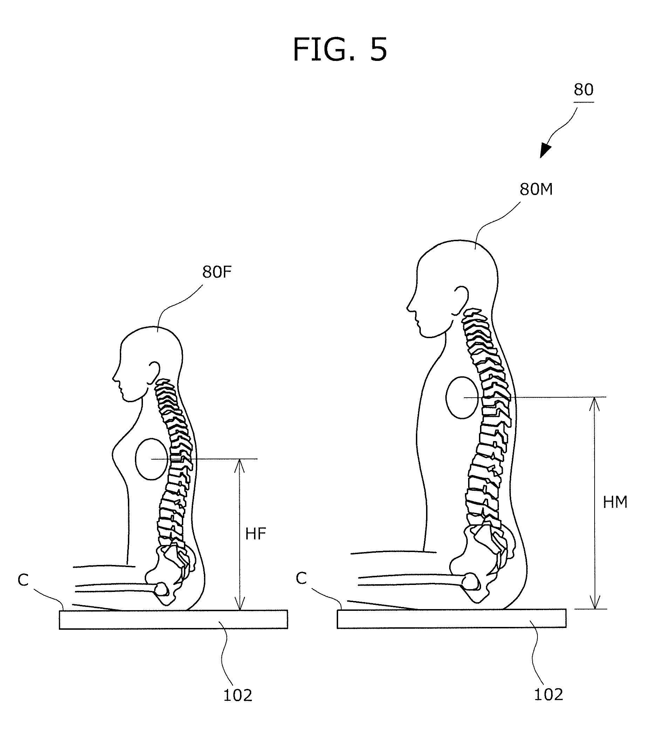

As shown in FIG. 1, a vehicle seat 10A according to the embodiment includes a seat cushion 102 that is a region on which a seated person 80 shown in FIG. 5 and described later is seated, a seat back 104 that is a region rotatably attached to a back portion of the seat cushion 102 (hereinafter, a vehicle forward direction will be referred to as "front direction" and an opposite direction to the vehicle forward direction will be referred to as "back direction") and that abuts on a backrest of the seated person 80, and a heartbeat measuring device 12 provided in the seat back 104 and including a sensor group 20 to be described later.

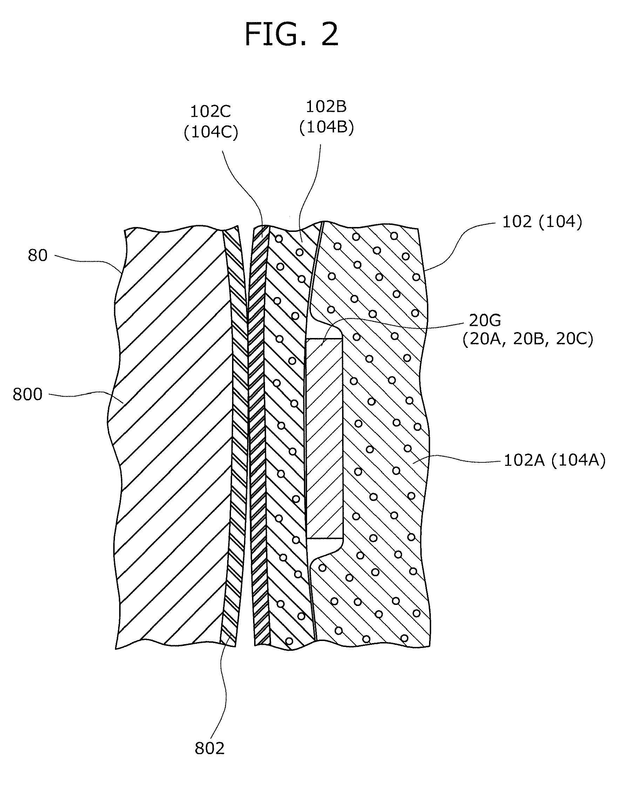

As shown in FIG. 2, the seat cushion 102 includes a urethane pad 102A, a urethane slab 102B adhesively bonded to an upper surface of the urethane pad 102A, and a skin 102C provided to further cover the urethane slab 102B. Furthermore, a ground electrode 20G, to be described later, is provided at a position facing the buttocks of the seated person 80 between the urethane pad 102A and the urethane slab 102B.

As shown in FIG. 2, the seat back 104 includes a urethane pad 104A, a urethane slab 104B adhesively bonded to an upper surface of the urethane pad 104A, and a skin 104C provided to further cover the urethane slab 104B. Sensors 20A to 20C, to be described later, are provided in the seat back 104 near on a surface of the seat back 104 (hereinafter, "front surface") facing the back of the seated person 80 between the urethane pad 104A and the urethane slab 104B.

As shown in FIG. 1, the heartbeat measuring device 12 includes the sensor group 20 provided in the seat back 104, the ground electrode 20G, an instrumentation amplifier 30, a DC-component elimination circuit 40, an inverting amplifier 50, a bandpass filter 60, an A/D converter circuit 62, an arithmetic operation device 70C, and a display D.

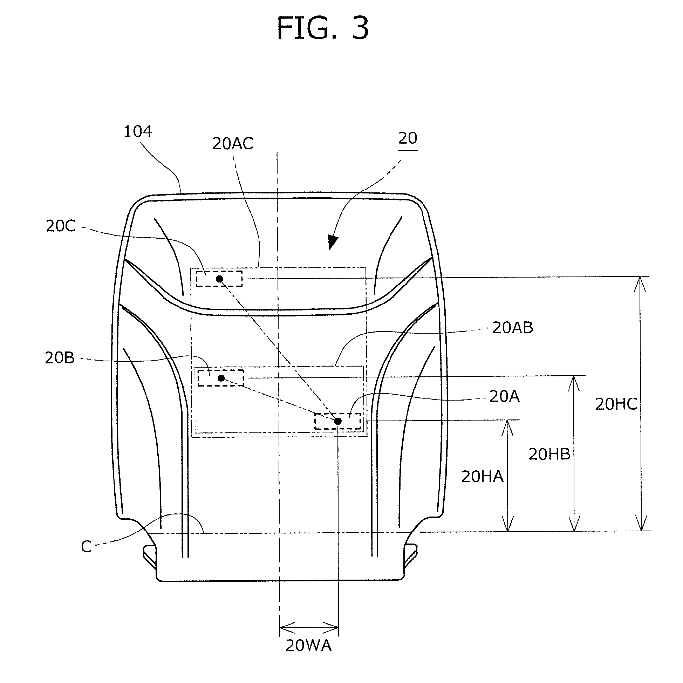

As shown in FIG. 3, the sensor group 20 is an assembly constituted by the sensors 20A, 20B, and 20C each formed out of a conductive fabric tape, and is provided in the seat back 104 near the front surface of the seat back 104. As shown in FIG. 2, each of the sensors 20A, 20B, and 20C functions to detect a body potential of the seated person 80 by being capacitively coupled to a body 800 of the seated person 80 via the skin 104C and clothes 802. A size of each of the sensors 20A, 20B, and 20C is 25 mm in height, 75 mm in width, and 1 mm in thickness. The sensors 20A, 20B, and 20C are arranged in the seat back 104 so that a longitudinal direction of the sensors 20A, 20B, and 20C is parallel to a width direction of the seat back 104. By so arranging, the seat back 104 can be downsized in a height direction.

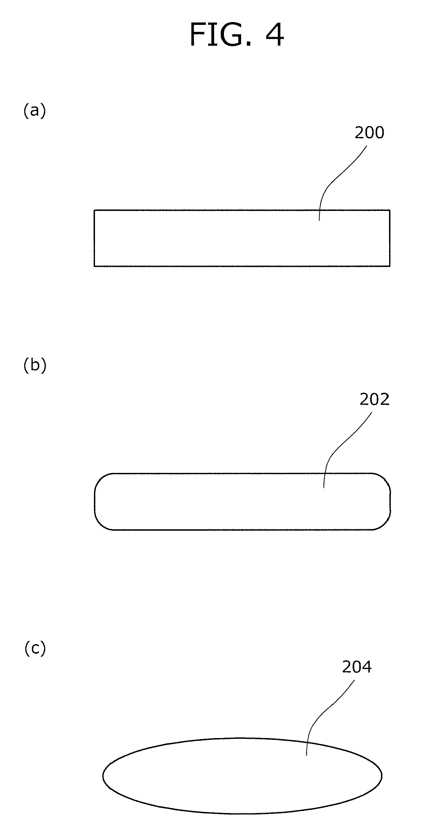

It suffices that a body-potential detection surface of each of the sensors 20A, 20B, and 20C has a shape having shorter and longer sides. The body-potential detection surface may be a rectangular detection surface 200 as shown in FIG. 4 (a), a chamfered rectangular detection surface 202 as shown in FIG. 4 (b) or an elliptic detection surface 204 as shown in FIG. 4 (c) . The arrangement of the sensors 20A, 20B, and 20C in the seat back 104 in the height and width directions thereof will be described later in detail.

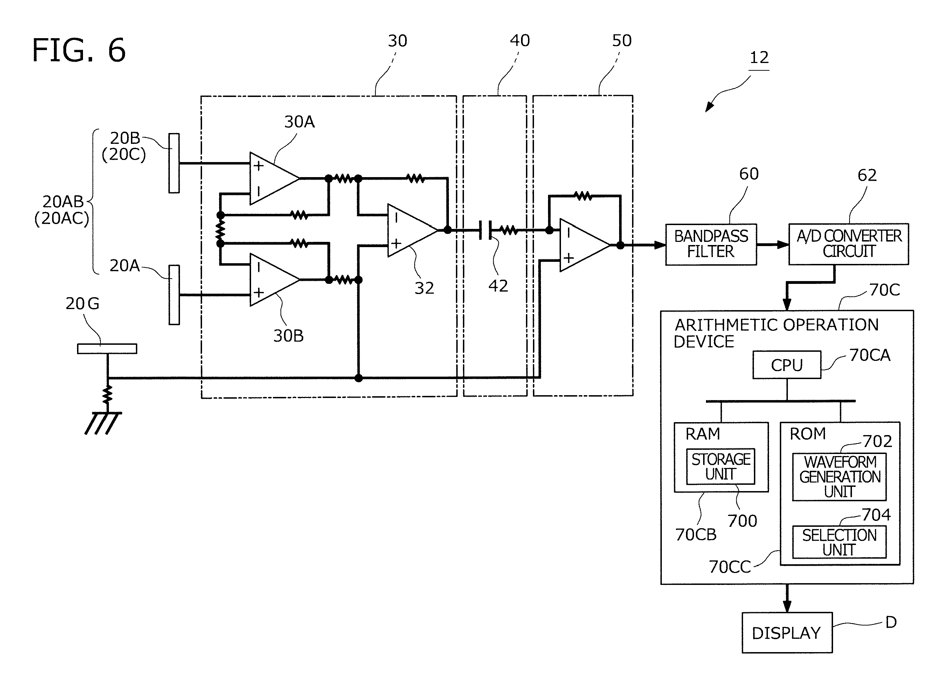

The ground electrode 20G is formed out of a conductive fabric tape and provided in a region of the seat cushion 102 facing the buttocks of the seated person 80. The ground electrode 20G acquires a potential that serves as a reference potential used when offset signals contained in the signals from the sensors 20A, 20B, and 20C are eliminated. Similarly to the sensors 20A, 20B, and 20C, the ground electrode 20G is capacitively coupled to the body 800 of the seated person 80 via the skin 104C and the clothes 802. Moreover, as shown in FIG. 6, the ground electrode 20C is connected to plus-side input terminals of an operational amplifier 32 and the inverting amplifier 50 via a resistor using a ground electrode cable.

As shown in FIG. 6, the instrumentation amplifier 30 is constituted by operational amplifiers 30A, 30B, and 32. The operation amplifiers 30A and 30B amplify potential signals detected by the sensor group 20 and output the amplified potential signals to the operational amplifier 32. FIG. 6 particularly and only shows a circuit configuration including the sensors 20A and 20B. Other sensors shown later are the same circuit configuration, for example, the circuit configuration including the sensors 20A and 20B is a configuration in which the sensor 20B is replaced by the sensor 20C in FIG. 6.

The operational amplifier 32 is constituted by a differential amplifier and amplifies a difference signal between the potential signals output from the operational amplifiers 30A and 30B.

The potential of the ground electrode 20G is applied to the plus-side input terminal of the operational amplifier 32. As described above, the ground electrode 20G is provided in the seat cushion 102 farther from the heart of the seated person 80 than the other sensors provided in the seat back 104, and capacitively coupled to the buttocks of the seated person 80. The ground electrode 20G, therefore, can acquire the potential less influenced by electrocardiographic signals.

A capacitor 42 acting as the DC-component elimination circuit 40 functions to eliminate low frequency components including a direct-current (DC) component and contained in the difference signal output from the operational amplifier 32, and provides AC coupling of an output terminal of the operation amplifier 32 to a minus-side input terminal of the inverting amplifier 50.

The inverting amplifier 50 functions to invert the polarity of the potential difference signal from which the DC component is eliminated and to further amplify the resultant potential difference signal. The minus-side input terminal of the inverting amplifier 50 is connected to the capacitor 42 via a resistor, and the potential of the ground electrode 20G is applied to the plus-side input terminal of the inverting amplifier 50 as the reference potential.

The bandpass filter 60 is provided to eliminate the low frequency components and high frequency components that are not regarded as frequencies of the electrocardiographic signals. By providing the bandpass filter 60, signals with frequencies relating to the electrocardiographic signals are input to the A/D converter circuit 62 in a restricted manner.

The A/D converter circuit 62 converts an analog signal input from the inverting amplifier 50 via the bandpass filter 60 to a digital signal so as to use the digital signal as an input signal to the arithmetic operation device 70C.

The arithmetic operation device 70C includes an arithmetic control CPU (Central Processing Unit) 70CA, a RAM (Random Access Memory) 70CB, and a ROM (Read Only Memory) 70CC. Furthermore, the signal input to the arithmetic operation device 70C is the potential difference signal that is the digital signal obtained by conversion in the A/D converter circuit 62, and a signal output from the arithmetic operation device 70C is an electric signal to be displayed on the display D.

The RAM 70CB temporarily stores therein parameters including signals under an arithmetic operation control and input/output signals. The RAM 70CB functions as a storage unit 700 that stores therein the digital-signal-converted potential difference signal and the other signals.

The ROM 70CC stores therein programs executed by the CPU 70CA and predetermined parameters. A waveform generation unit 702 that generates voltage waveform data from the potential difference signal obtained from the sensor group 20 and a selection unit 704 that selects voltage waveform data having periodic amplitude corresponding to heart contraction are recorded in the ROM 70CC as the programs.

The waveform generation unit 702 functions to generate voltage waveform data VAB and VAC with a vertical axis representing the potential difference signal and a horizontal axis representing time based on the potential difference signal between the potential signals output from the sensors 20A and 20B that is stored in the storage unit 700 and the potential difference signal between the potential signals output from the sensors 20A and 20C.

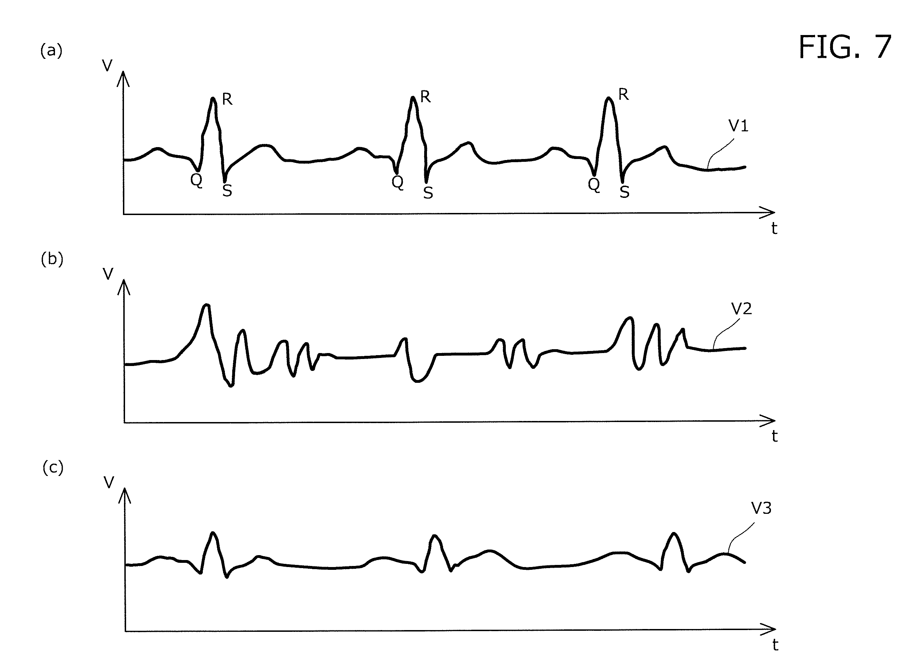

The selection unit 704 functions to select the voltage waveform data corresponding to the heart contraction from between the voltage waveform data VAB and VAC, and to set the selected voltage waveform data as electrocardiographic waveform data VH. For example, a case is assumed where waveform data V1 shown in FIG. 7 (a) is generated as the voltage waveform data VAB, and where waveform data V2 shown in FIG. 7(b) or waveform data V3 shown in FIG. 7(c) is generated as the voltage waveform data VAC. In this case, the selection unit 704 selects the voltage waveform data V1 on which periodic R-waves clearly appear and that has high amplitude, that is, the voltage waveform data VAB.

The arrangement of the sensor group 20 for stably detecting body potentials irrespectively of a physical difference between the seated people 80 will be described. In the following description, an upper surface of the seat cushion 102 abutting on lower ends of the buttocks is defined as a cushion seat surface C in a state in which the seated person 80 (each of a female 80F and a male 80M) is seated on the seat cushion 102, and the cushion seat surface C is used as a reference for clearly expressing heights.

First, the seated people 80 having a physical difference are defined as follows. FIG. 5 shows the female 80F as the seated person 80 having a small build and the male 80M as the seated person 80 having a large build, for the physically different seated people 80. The female 80F assumed as the seated person 80 having the small build is, for example, one of about 150-cm-tall Japanese female adults who are 5% of a total number accumulated from a shorter side. Furthermore, the male 80M assumed as the seated person 80 having the large build is, for example, one of about 190-cm-tall American male adults who are 95% of a total number accumulated from a shorter side.

The heart of the female 80F is at a position of a height HF from the cushion seat surface C whereas the heart of the male 80M is at a position of a height HM from the cushion seat surface C. This height HF is about 370 mm for the female 80F who is about 150 cm tall whereas the height HM is about 550 mm for the male 80M who is about 190 cm tall. The following description will be given while using the female 80F as an expression replacing the seated person 80 having the small build and the male 80M as an expression replacing the seated person 80 having the large build.

The arrangement of the sensors 20A, 20B, and 20C in the seat back 104 in the height and width directions thereof will be described. The sensor group 20 is arranged depending on the difference in the position of the heart due to the physical difference. Specifically, as shown in FIG. 3, the sensor 20A placed in a lower portion among the three sensors is arranged so that a height 20HA of a center of the sensor 20A from the cushion seat surface C is smaller than the height HF of the heart of the female 80F. Moreover, the sensor 20A is provided offset rightward from a central axis by as much as a width 20WA while facing the front surface of the seat back 104. This width 20WA is arbitrarily set within a range from about 55 mm to about 75 mm.

Furthermore, the sensor 20C placed in an upper portion among the three sensors is arranged so that a height 20HC of a center of the sensor 20C from the cushion seat surface C is larger than the height HM of the heart of the male 80M. Moreover, the sensor 20C is provided offset leftward from the central axis while facing the front surface of the seat back 104.

Furthermore, the sensor 20B placed in an intermediate portion among the three sensors is arranged so that a height 20HB of a center of the sensor 20B from the cushion seat surface C is larger than the height HF of the heart of the female 80F. Moreover, the sensor 20B is provided on the left and at the same position as the sensor 20C in the width direction while facing the front surface of the seat back 104. Note that the a length outward in a direction of a width between the sensors 20A and 20B, that is, a width of a zone 20AB is about 250 mm.

In the sensor group 20 arranged as described above, the upper left sensor 20C and the lower right sensor 20A, which face the front surface of the seat back 104, are arranged to obliquely hold the heart of the male 80M therebetween. An area defined as having the sensor 20A as a lower right corner and the sensor 20C as an upper left corner is assumed as a zone 20AC. This zone 20AC is the area that makes it possible to obtain a suited potential difference for detecting a potential of the heart of the male 80M. A heartbeat measuring method will be described later.

Furthermore, the upper left sensor 20B and the lower right sensor 20A, which face the front surface of the seat back 104, are arranged to obliquely hold the heart of the female 80F therebetween. An area defined as having the sensor 20A as a lower right corner and the sensor 20B as an upper left corner is assumed as a zone 20AB. This zone 20AB is the area that makes it possible to obtain a suited potential difference for detecting a potential of the heart of the female 80F.

The reason for arranging the sensors 20A, 20B, and 20C as described above is as follows. A vector of an electromotive force of the heart generated during expansion and contraction of the heart is generally in a direction from the right shoulder to the left leg if the seated person 80 is an ordinary person. This direction corresponds to a direction from upper left to lower right when facing the front surface of the seat back 104 in a state in which the seated person 80 is seated on the vehicle seat 10A. Therefore, the sensors 20A, 20B, and 20C are arranged so that a direction connecting the sensor 20A to the sensor 20B and that connecting the sensor 20A to the sensor 20C (indicated by two-dot chain lines in FIG. 3) are along the direction of the vector of the electromotive force of the heart. For this reason, a combination of the sensors 20A and 20B or that of the sensors 20A and 20C can be used to detect a large potential difference signal generated in response to the heart contraction (potential difference signal corresponding to the R-waves in an electrocardiogram).

### (Heartbeat measuring process)

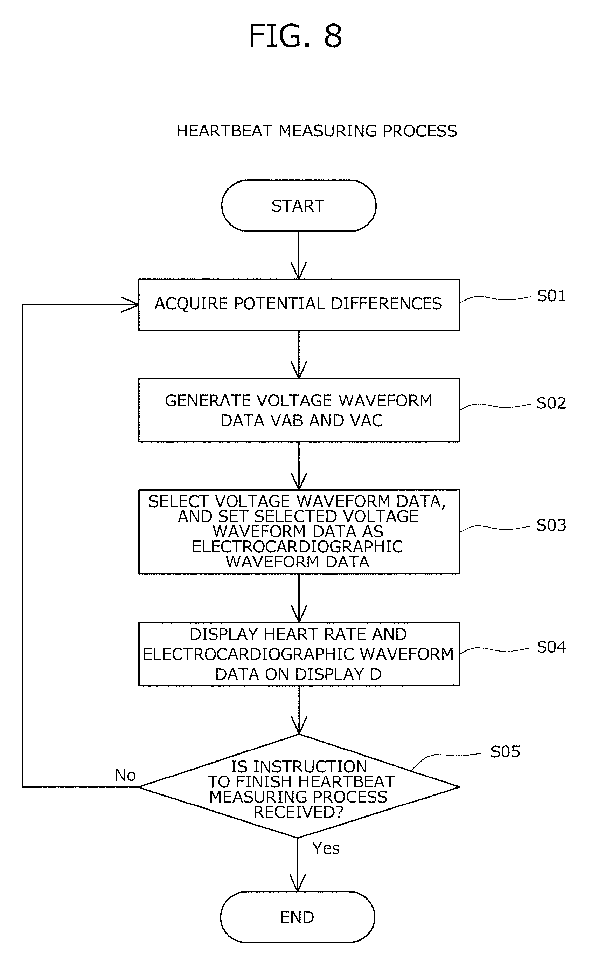

A heartbeat measuring process performed by the heartbeat measuring device 12 configured as described above will be described with reference to FIG. 8. The sensors 20A, 20B, and 20C detect the potential signals from the body 800 in response to start of a vehicle engine or to depression of a start switch.

The potential signals detected by the sensors 20A and 20B and the sensor 20A and 20C are stored in the storage unit 700 of the arithmetic operation device 70C via the instrumentation amplifier 30, the DC-component elimination circuit 40, the inverting amplifier 50, the bandpass filter 60, and the A/D converter circuit 62 as the potential difference data. That is, the arithmetic operation device 70C acquires the potential difference data in the zones 20AB and 20AC (Step S01).

Based on the potential difference data acquired in the zones 20AB and 20AC, the waveform generation unit 702 generates the two voltage waveform data VAB and VAC with the respective axes representing the potential difference and the time (Step S02).

The selection unit 704 selects the data synchronous with heartbeats and having the high amplitude of the R-waves from between the two voltage waveform data VAB and VAC, and sets the selected data as the electrocardiographic waveform data VH (Step S03).

The arithmetic operation device 70C computes a peak interval at which a voltage (R-wave potential) exceeding a set threshold is detected by digitally filtering the electrocardiographic waveform data VH for emphasizing a waveform corresponding to QRS waves. Next, the arithmetic operation device 70C sets an inverse of the peak interval as an instantaneous heart rate (heart rate per second) and further calculates the number of heartbeats detected per minute. That is, the arithmetic operation device 70C calculates the heart rate by arithmetic operation. Next, the arithmetic operation device 70C transmits signals relating to the electrocardiographic waveform data VH and the heart rate to the display D so as to display the electrocardiographic waveform data VH and the heart rate on the display D (Step S04).

The arithmetic operation device 70C determines whether an instruction to finish heartbeat measurement is received from a stop switch or the like (Step S05). If the arithmetic operation device 70C receives the instruction to finish the heartbeat measurement, the process ends. If the arithmetic operation device 70C does not receive the instruction to finish the heartbeat measurement, Steps S01 to S05 are repeated.

It is effective to set lower limit time that is inappropriate for an R-wave generation interval as an algorithm used for the arithmetic operation of the peak interval. Specifically, after detection of one R-wave, voltage signals detected at shorter intervals than this time are eliminated from a signal corresponding to a next R-wave. By doing so, it is possible to prevent the peak interval from being computed as a value that does not obviously correspond to a heart contraction cycle. Alternatively, if the peak interval exceeds an upper limit of a set range, for example, such a high voltage as to correspond to the voltage of the R-wave may be searched again at time near an intermediate point of the interval or the peak interval may be set as half of the time assuming that the R-wave is detected at the intermediate point. By doing so, even if the voltage corresponding to the R-wave cannot be temporarily detected due to, for example, a body motion or the like of the seated person 80, it is possible to prevent the heart rate that is obviously erroneous from being output.

As described above, in the vehicle seat 10A according to the embodiment, the upper left sensor 20C and the lower right sensor 20A, which face the front surface of the seat back 104, are arranged so as to obliquely hold the heart of the male 80M therebetween. Furthermore, the upper left sensor 20B and the lower right sensor 20A, which face to the front surface of the seat back 104, are arranged so as to obliquely hold the heart of the female 80F therebetween. In the zone 20AC defined by the sensors 20A and 20C, the suited potential difference signal corresponding to the potential of the heart of the male 80M having the large build can be acquired. In the zone 20AB defined by the sensors 20A and 20B, the suited potential difference signal corresponding to the potential of the heart of the female 80F having the small build can be acquired.

Moreover, the selection unit 704 of the arithmetic operation device 70C selects the voltage waveform data synchronous with the heartbeats and having the high amplitude from between the voltage waveform data VAB and VAC obtained in the zones 20AB and 20AC, and sets the selected voltage waveform data as the electrocardiographic waveform data VH. It is, therefore, possible to stably set the voltage waveform data containing the QRS waves as the electrocardiographic waveform data VH whoever is seated, the male 80M or the female 80F.

### (First Modification)

A sensor group 21 according to a first modification will next be described. In the following description, for making clearer the difference in features of the first modification from the embodiment, the same contents as those of the vehicle seat 10A according to the embodiment are not repeatedly described.

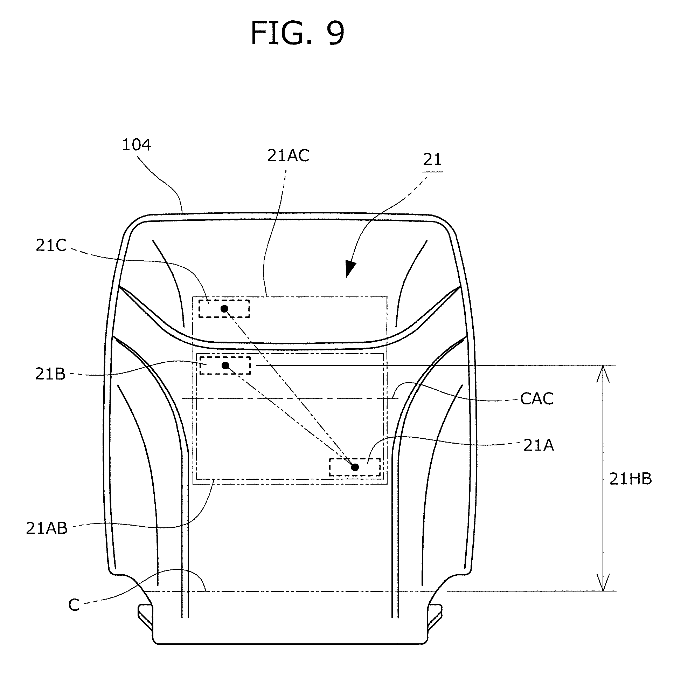

As shown in FIG. 9, the sensor group 21 according to the first modification is an assembly provided in the seat back 104 near the front surface of the seat back 104 and constituted by sensors 21A, 21B, and 21C arranged from below in order. Note that the arrangement of the sensors 21A and 21C is identical to that of the sensors 20A and 20C according to the embodiment. It is assumed herein that an area defined as having the sensor 21A as a lower right corner and the sensor 21C as an upper left corner is a zone 21AC, and that an area defined as having the sensor 21A as a lower right corner and the sensor 21B as an upper left corner is a zone 21AB. Naturally, this zone 21AC is the area identical to the zone 20AC.

The sensor group 21 according to the first modification differs from the sensor group 20 according to the embodiment as follows. A height 21HB of a center of the sensor 21B from the cushion seat surface C is larger than a height of a centerline CAC of the zone 21AC extending in a width direction of the zone 21AC. By so arranging the sensor 21B and forming the zone 21AB wider than the zone 20AB, it is possible to detect a large potential difference from the sensors 21A and 21B if the seated person 80 is the female 80F.

### (Second Modification)

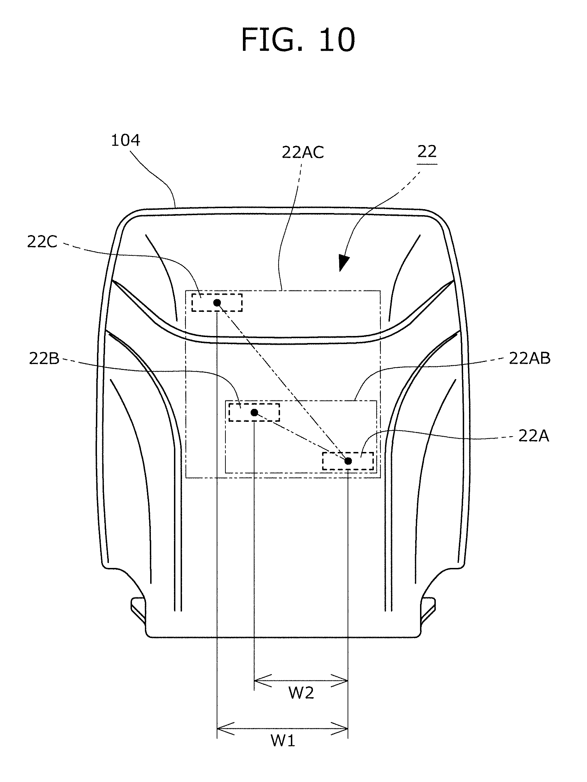

As shown in FIG. 10, a sensor group 22 according to a second modification is an assembly provided in the seat back 104 near the front surface of the seat back 104 and constituted by sensors 22A, 22B, and 22C arranged from below in order. Note that the arrangement of the sensors 22A and 22C is identical to that of the sensors 20A and 20C according to the embodiment. It is assumed herein that an area defined as having the sensor 22A as a lower right corner and the sensor 22C as an upper left corner is a zone 22AC, and that an area defined as having the sensor 22A as a lower right corner and the sensor 22B as an upper left corner is a zone 22AB. Naturally, this zone 22AC is the area identical to the zone 20AC as well.

The sensor group 22 according to the second modification is characterized in that a width W1 between centers of the sensors 22A and 22C is larger than a width W2 between centers of the sensors 22A and 22B.

Because of the physical difference between the male 80M and the female 80F, the male 80M and the female 80F differ in a contact height and a contact width with the seat back 104. That is, a detection range of the potential difference of the male 80M is preferably set wider than that of the female 80F so as to acquire a high potential difference in a contact range limited by the seated person 80 of each physical constitution. With a configuration including the sensor group 22 according to the second modification, it is possible to detect the potential difference suited for the female 80F in the zone 22AB and the potential difference suited for the male 80M in the zone 22AC.

### (Third Modification)

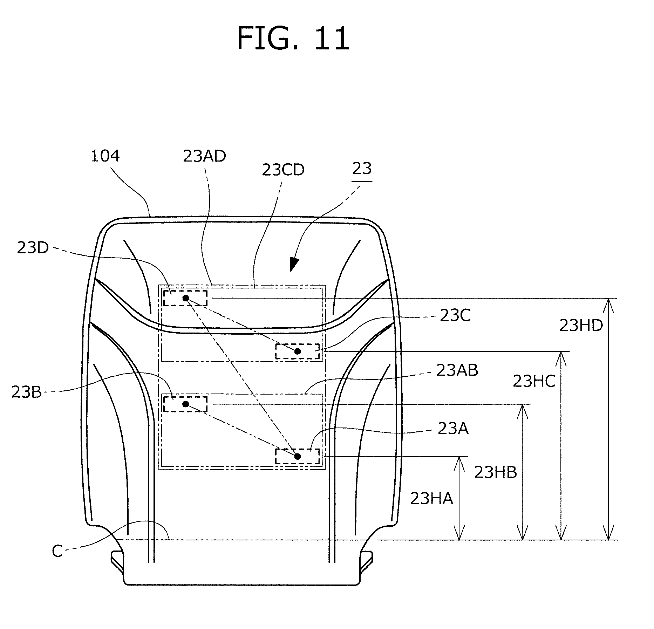

As shown in FIG. 11, a sensor group 23 according to a third modification is an assembly provided in the seat back 104 near the front surface of the seat back 104 and constituted by four sensors 23A, 23B, 23C, and 23D arranged from below in order. Note that the arrangement of the sensors 23A, 23B, and 23D is identical to that of the sensors 20A, 20B, and 20C according to the embodiment. Naturally, heights 23HA, 23HB, and 23HD of the respective sensors 23A, 23B, and 23D in the seat back 104 from the cushion seat surface C are identical to the heights 20HA, 20HB, and 20HC of the respective sensors 20A, 20B, and 20C.

The sensor 23C is provided to detect a potential signal containing QRS-waves of the male 80M. Owing to this, the sensor 23C is arranged above the sensor 23B and below the sensor 23D, and arranged vertically above the sensor 23A. The sensor 23C is arranged so that the heart of the male 80M is held obliquely between the sensors 23C and 23D.

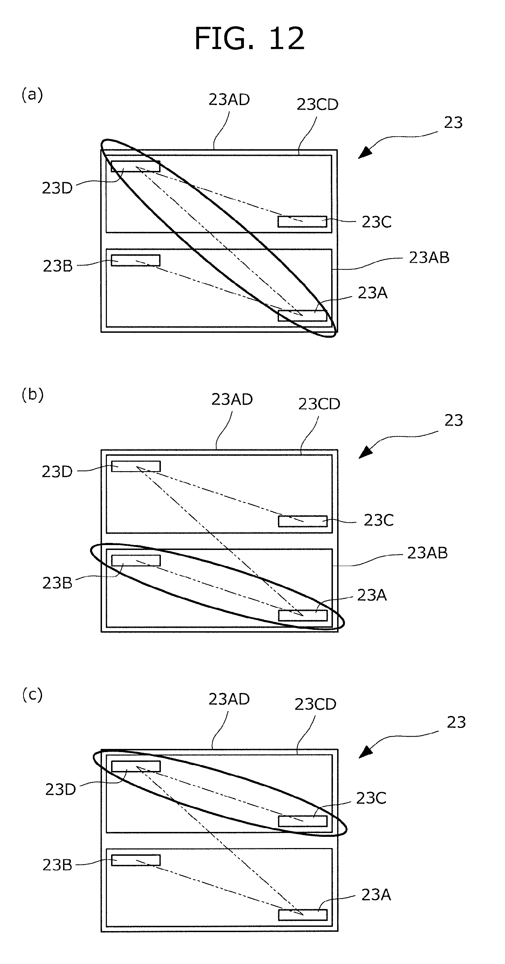

As shown in FIGS. 12 (a) to 12 (c), a zone 23AB is an area defined as having the sensor 23A as a lower right corner and the sensor 23B as an upper left corner. A zone 23AD is an area defined as having the sensor 23A as a lower right corner and the sensor 23D as an upper left corner. A zone 23CD is an area defined as having the sensor 23C as a lower right corner and the sensor 23D as an upper left corner. The zone 23AB is the area identical to the zone 20AB and the zone 23AD is the area identical to the zone 20AC.

A circuit configuration relating to the sensor group 23 constituted by the four sensors 23A, 23B, 23C, and 23D will be described with reference to FIG. 6. The zone 23AB is configured so that the sensor 20A is replaced by the sensor 23A and the sensor 20B is replaced by the sensor 23B. Likewise, the zone 23AD is configured so that the sensor 20A is replaced by the sensor 23A and the sensor 20B is replaced by the sensor 23D. Likewise, the zone 23CD is configured so that the sensor 20A is replaced by the sensor 23A and the sensor 20B is replaced by the sensor 23D.

In the sensor group 23 configured as described above, the two sensors 23A and 23C are combined with the sensor 23D each for detecting a potential difference. The sensor group 23 with such a configuration can detect a large potential difference if the seated person 80 is the male 80M having a vector of an electromotive force of the heart eccentric to a left axis. The sensor group 23 has more voltage waveform data to be selected by the selection unit 704 than the sensor group 21 by as much as the voltage waveform data detected by the combination of the sensors 23C and 23D. The heartbeat measuring device 12 can, therefore, obtain the electrocardiographic waveform data VH containing the QRS-waves more stably.

### (Fourth Modification)

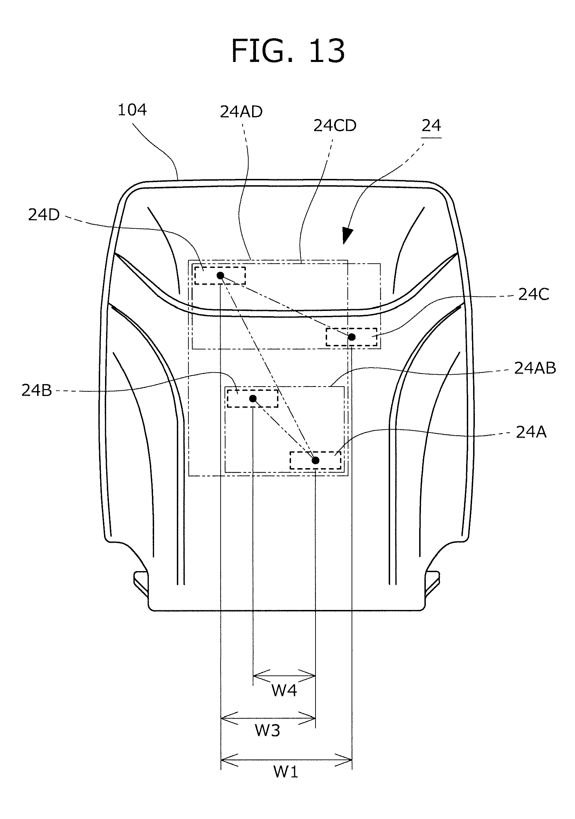

As shown in FIG. 13, a sensor group 24 according to a fourth modification is an assembly provided in the seat back 104 near the front surface of the seat back 104 and constituted by four sensors 24A, 24B, 24C, and 24D arranged from below in order. Note that heights of the sensors 24A, 24B, 24C, and 24D are identical to those of the sensors 23A, 23B, 23C, and 23D according to the third modification, respectively. The arrangement of the sensors 24C and 24D is identical to that of the sensors 23C and 23D according to the third modification. Similarly to the sensor 20A shown in FIG. 3, the sensor 24A is provided at a position offset from the central axis of the seat back 104 by as much as the width 20WA.

A width W3 between centers of the sensors 24A and 24D is smaller than a width W1 between centers of the sensors 24C and 24D. A width W4 between centers of the sensors 24A and 24B is smaller than the width W3. In other words, the sensor 24A is arranged closer to a center of the seat back 104 in the width direction than the sensor 24C, and the sensor 24B is arranged closer to the center of the seat back 104 in the width direction than the sensor 24D.

Furthermore, a zone 24AB is an area defined as having the sensor 24A as a lower right corner and the sensor 24B as an upper left corner. A zone 24AD is an area defined as having the sensor 24A as a lower right corner and the sensor 24D as an upper left corner. A zone 24CD is an area defined as having the sensor 24C as a lower right corner and the sensor 24D as an upper left corner. The zone 24CD is the area identical to the zone 23CD.

The sensor group 24 configured as described above can detect a large potential difference if the seated person 80 is the male 80M having the vector of the electromotive force of the heart eccentric to the left axis similarly to the sensor group 23 according to the third modification. Moreover, the sensor group 24 according to the fourth modification can detect a suited potential difference for the female 80F having a small body width in the zone 24AB, and detect a suited potential difference for the male 80M having a large body width in each of the zones 24AD and 24CD, thus providing sensor arrangements suited for physical constitutions.

### (Fifth Modification)

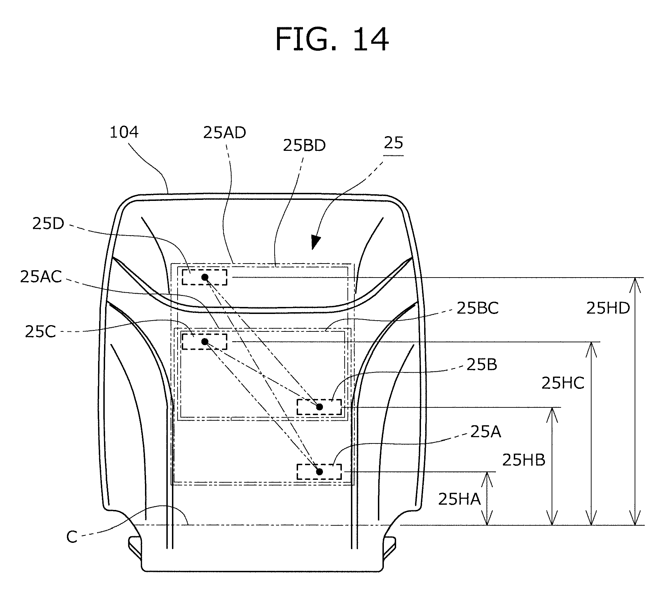

As shown in FIG. 14, a sensor group 25 according to a fifth modification is an assembly provided in the seat back 104 near the front surface of the seat back 104 and constituted by four sensors 25A, 25B, 25C, and 25D arranged from below in order. Note that the arrangement of the sensors 25A and 25D is identical to that of the sensors 24A and 24D according to the fourth modification. Similarly to the sensor 20A shown in FIG. 3, the sensors 25A and 25D are at positions of heights 25HA and 25HD from the cushion seat surface C to be offset from the central axis of the seat back 104 by as much as the width 20WA.

The sensors 25B and 25C are arranged axisymmetric to the sensors 23B and 23C according to third modification about a centerline of the seat back 104 extending in the height direction.

Specifically or in other words, the sensor 25B is arranged in the seat back 104 so as to have a height 25HB identical to the height 23HB of the sensor 23B according to the third modification from the cushion seat surface C. The sensor 25C is arranged in the seat back 104 so as to have a height identical to the height 23HC of the sensor 23C according to the third modification from the cushion seat surface C. That is, the sensor 25C is arranged above the sensor 25B in the seat back 104. Furthermore, the sensor 25B is provided on a vertical line of the sensor 25A in the seat back 104, and the sensor 25C is provided on a vertical line of the sensor 25D.

Moreover, the sensor 25C is arranged in the seat back 104 so as to obliquely hold the heart of the female 80F and the heart of the seated person 80 of an intermediate build that is intermediate between those of the female 80F and the male 80M between the sensors 25C and 25A. The sensor 25B is arranged so as to obliquely hold the heart of the male 80M and the heart of the seated person 80 of the intermediate build between the sensors 25B and 25D. Furthermore, the sensors 25B and 25C are arranged so as to obliquely hold the heart of the seated person 80 of the intermediate build therebetween.

It is assumed that an area defined as having the sensor 25A as a lower right corner and the sensor 25C as an upper left corner is a zone 25AC, and that an area defined as having the sensor 25A as a lower right corner and the sensor 25D as an upper left corner is a zone 25AD. Furthermore, it is assumed that an area defined as having the sensor 25B as a lower right corner and the sensor 25C as an upper left corner is a zone 25BC, and that an area defined as having the sensor 25B as a lower right corner and the sensor 25D as an upper left corner is a zone 25BD.

A circuit configuration relating to the sensor group 25 constituted by the four sensors 25A, 25B, 25C, and 25D will be described with reference to FIG. 6. The zone 25AC is configured so that the sensor 20A is replaced by the sensor 25A and the sensor 20B is replaced by the sensor 25C. Likewise, the zone 25AD is configured so that the sensor 20A is replaced by the sensor 25A and the sensor 20B is replaced by the sensor 25D. Likewise, the zone 25BC is configured so that the sensor 20A is replaced by the sensor 25B and the sensor 20B is replaced by the sensor 25C. Likewise, the zone 25BD is configured so that the sensor 20A is replaced by the sensor 25B and the sensor 20B is replaced by the sensor 25D.

In the sensor group 25 configured as described above, the two sensors 25A and 25B are combined with the sensor 25D each for detecting a potential difference. Furthermore, the two sensors 25A and 25B are combined with the sensor 25C each for detecting a potential difference. The sensor group 25 with such a configuration can detect a large potential difference if the seated person 80 is a person of the intermediate build.

The sensor 25B is arranged at the lower right position relatively to the sensor 25C while facing the front surface of the seat back 104. That is, the sensors 25B and 25C are arranged in a direction along the vector of the electromotive force of the heart. A combination of the sensors 25B and 25C can, therefore, detect a high potential difference. Moreover, the sensor group 25 has more voltage waveform data to be selected by the selection unit 704 than the sensor group 23 by as much as the voltage waveform data detected by the combination of the sensors 25B and 25C. The heartbeat measuring device 12 can, therefore, obtain the electrocardiographic waveform data VH containing the QRS-waves more stably.

### (Sixth Modification)

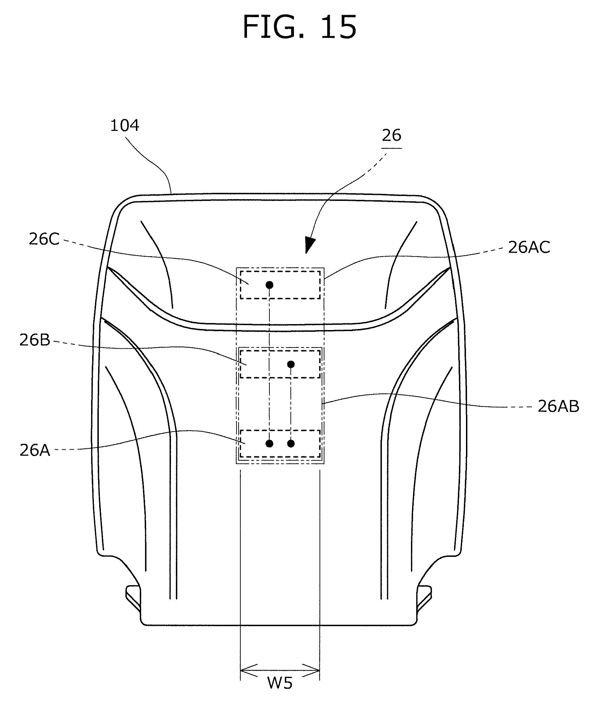

As shown in FIG. 15, a sensor group 26 according to a sixth modification is an assembly provided in the seat back 104 near the front surface of the seat back 104 and constituted by sensor units 26A, 26B, and 26C arranged in a line in a vertical direction from below in order.

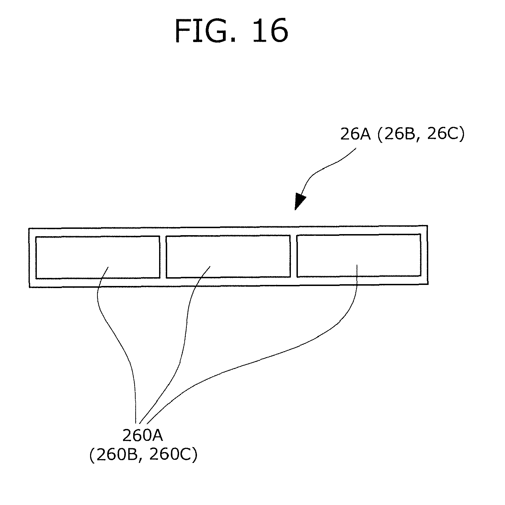

As shown in FIG. 16, the sensor units 26A, 26B, and 26C are constituted by three capacitively coupled sensors 260A, 260B, and 260C formed out of conductive fabric tapes, respectively, and arranged on a centerline extending vertically from the seat back 104.

Heights of the sensor units 26A, 26B, and 26C in the seat back 104 from the cushion seat surface C are identical to the heights 20HA, 20HB, and 20HC of the sensors 20A, 20B, and 20C, respectively. That is, the sensor units 26A and 26B are provided so that the heart of the female 80F is located in a zone 26AB that is an area defined by positions of the sensor units 26A and 26B. Likewise, the sensor units 26A and 26C are provided so that the heart of the male 80M is located in a zone 26AC that is an area defined by positions of the sensor units 26A and 26C.

That is, the sensor group 26 is arranged so as to hold the heart of each of the male 80M and the female 80F between any one of the sensors 260B or 260C located at an upper left position and any one of the sensors 260A located at a lower right position while facing the front surface of the seat back 104.

Even with such a configuration, it is possible to stably detect the potential of the heart irrespectively of the physical constitution by selecting the data on which the QRS-waves appear most clearly from among the voltage waveform data acquired by combinations of the sensors 260A, 260B, and 260C.

Moreover, with such a configuration, even if the body of the seated person 80 is offset to one side in the width direction of the seat by a centrifugal force generated when the vehicle driven by the seated person 80 makes a turn, the contact of the seated person 80 with the sensor group 26 is easily maintained and the sensor group 26 can stably acquire the potential difference.

The sensors provided in the vehicle seat 10A according to the embodiment and the sensors according to the modifications other than the embodiment may be configured similarly to the sensor units 26A, 26B, and 26C according to the sixth modification. In other words, each sensor may be a sensor unit constituted by a plurality of sensors.

Furthermore, each of the sensor units 26A, 26B, and 26C may be constituted by one sensor. Even with such a configuration, each of the sensor units 26A, 26B, and 26C can detect a large potential difference if the seated person has the vector of the electromotive force of the heart eccentric to the right axis. In this case, each of the sensor units 26A, 26B, and 26C preferably has a width W5 larger than the width of the sensor unit 20A since the zones 26AB and 26AC are limited in the width of the sensor.

While the present invention has been described specifically based on the embodiment and the modifications thereof, the present invention is not limited to the embodiment and the modifications. Various changes such as a combination of the respective features and the like can be made of the invention within the scope of the spirit of the invention.

For example, while the number of sensors is three or four in the vehicle seat according to the embodiment and the modifications thereof, the number of sensors is not limited to this number but more plural sensors may be provided in light of the balance between the stability of the detection of the potential difference and manufacturing cost.

While it has been described that the sensors constituting the sensor groups and the ground electrodes are each formed out of the conductive fabric tape, the material may be a metal conductor having conduction, for example, conductive fiber and the like.

While it has been described that this heartbeat measuring device includes the display for displaying electrocardiographic waveforms, the device may also include a vibrating motor that is equipment for keeping the seated person awake, or an oscillator producing an alarm or a light-emitting unit emitting light as well as the display.

Moreover, the vehicle seat according to the embodiment is applicable for the purpose of monitoring a person who has heart disease. In this case, the display is preferably arranged at a position at which another passenger can monitor the operating state of the heart of the person with disease. Alternatively, the vehicle seat may be configured so that a seat (seats) other than the seat provided with the heartbeat measuring device includes (include) a vibrating motor that can vibrate the other seat (seats) to notify the other passenger (passengers) of the functional deterioration of the heart of the person having heart disease if the functional deterioration is detected.

While it has been described above that the seated person assumed to have the small build is the 150-cm-tall person and the seated person assumed to have the large build is the 190-cm-tall person by way of example, the seated person of the small build and the seated person of the large build can be arbitrarily assumed. For example, in a case where only American adults are assumed to be seated or only children are assumed to be seated, the seated people are set based on the physical constitution according to the assumption. By determining the positions of the upper left and lower right sensors, which face the front surface of the seat back, so as to hold the heart of the person set as a basis therebetween and determining the position of another sensor therebetween in a state in which the person of the physical constitution as the basis is seated, similar effects can be attained.

Moreover, the vehicle seat according to the present invention can easily detect electrocardiographic signals without need to directly attach a sensor to the body of the seated person since the vehicle seat includes the capacitively coupled sensors. It is also possible to measure heartbeats of the seated person other than the driver since no sensor is provided in the steering wheel.

It is assumed that examples of the vehicle seat according to the present invention include not only seats of a motorbike and a motor scooter that are land-based vehicles but also those of a snowmobile and a personal watercraft, as well as vehicle seats of a straddle type vehicle such as a three-wheeled buggy or the like and a vehicle seat relating to a construction machine seat.

### Reference Numerals

- 10A: Vehicle seat
- 102: Seat cushion
- 104: Seat back
- 12: Heartbeat measuring device
- 20, 21, 22, 23, 24, 25, 26: Sensor group
- 20A, 21A, 22A, 23A, 24A, 25A, 260A: Sensor (first sensor)
- 20B, 21B, 22B, 23C, 24C, 25C, 260B: Sensor (second sensor)
- 20C, 21C, 22C, 23D, 24D, 25D, 260C: Sensor (third sensor)
- 23B, 24B, 25B: (Fourth sensor)
- 26A, 26B, 26C: Sensor unit
- 20AC, 21AC, 22AC, 23AD, 24AD, 25AD, 26AC: Zone (first zone)
- 20AB, 21AB, 22AB, 23AB, 24AB, 25AC, 26AB: Zone (second zone)
- 23CD, 24CD, 25BD: Zone (third zone)
- 25BC: Zone (fourth zone)
- 70C: Arithmetic operation device
- 700: Storage unit
- 702: Waveform generation unit
- 704: Selection unit
- 80: Seated person
- 80M: Male
- 80F: Female
- C: Cushion seat surface
- D: Display

## Claims

1. A vehicle seat comprising:
a seat back on which a seated person leans; and
a measuring device comprising a plurality of sensors that are capacitively coupled sensors acquiring body potentials of the seated person in a non-contact manner, and that are provided in the seat back, and an arithmetic operation device that computes electrical signals relating to the detected body potentials to obtain an electrocardiographic signal of the seated person, the measuring device measuring heartbeats of the seated person,
wherein the sensors are provided to face the seated person in a range from a lower portion to an upper portion in the seat back, and comprise at least three sensors of a first sensor provided in the lower portion, a second sensor provided in an intermediate portion, and a third sensor provided in the upper portion,
wherein the first sensor is arranged at a lower right position of a front surface of the seat back relatively to the second sensor and the third sensor when seen from a direction facing the front surface of the seat back, the front surface being a surface of the seat back facing the seated person,
wherein the second sensor is arranged above the first sensor and below the third sensor,
wherein the first sensor and the third sensor are arranged so that a heart of the seated person is located in a first zone if the first zone is defined based on a position of the first sensor and a position of the third sensor and a second zone is defined based on the position of the first sensor and a position of the second sensor, and
wherein the first sensor, the second sensor, and the third sensor transmit electrical signals at least relating to the body potentials detected in the first zone and the second zone to the arithmetic operation device.

2. The vehicle seat according to claim 1, wherein the second sensor is provided at the position of a height larger than a height of a center of the first zone.

3. The vehicle seat according to claim 1 or 2, wherein a length in a direction of a width between the first sensor and the third sensor is larger than a length in a direction of a width between the first sensor and the second sensor.

4. The vehicle seat according to any one of claims 1 to 3, wherein
the plurality of sensors further comprise a fourth sensor above the first sensor and below the third sensor, and
the first sensor, the second sensor, the third sensor, and the fourth sensor transmit the body potentials detected in the first zone, the second zone, and a third zone to the arithmetic operation device for computing the body potentials to obtain the electrocardiographic signal of the seated person if the third zone is defined based on the position of the third sensor and a position of the fourth sensor.

5. The vehicle seat according to any one of claims 1 to 4, wherein each of the first sensor, the second sensor, and the third sensor comprises a detection surface having shorter sides and longer sides, and is arranged so that a direction of the longer sides is along a width direction of the seat back.

6. The vehicle seat according to any one of claims 1 to 5, wherein the measuring device measures the heartbeats by selecting data having periodic amplitude corresponding to a heart contraction from among data detected by the plurality of sensors defining the zones.
